# EUROPEAN PATENT APPLICATION

(11) **EP 4 438 006 A1**
(43) Date of publication of application: **02.10.2024**
(21) Application number: 22899043.8
(22) Date of filing: 23.11.2022
(51) Int. Cl.: A61D 9/00, A61D 99/00

(54) **VAGUS NERVE STIMULATION DEVICE FOR ANIMALS**

(30) Priority: 26.11.2021 KR 20210165571
(71) Applicant: Neurive Co., Ltd., Juchon-myeon Gimhae-si, Gyeongsangnam-do 50969 (KR)
(72) Inventor: SONG, Jae Jun, Seoul 06501 (KR); CHOI, Hyuk, Seoul 06095 (KR); HONG, Ki Hwan, Seoul 03709 (KR)
(74) Representative: Franke, Dirk
(86) International application number: PCT/KR2022/018655
(87) International publication number: WO 2023/096361

(57) **Abstract**

The present invention relates to a vagus nerve stimulation device for animals, the device comprising an electrode that contacts the animal's skin to transmit electrical stimuli and a power source for generating electrical stimuli transmitted to the electrode. The present invention has an excellent effect of stabilizing the psychological state of an animal by stimulating the vagus nerve from the outside of the animal. For companion animals, the present invention has the effect of eliminating aggression, relieving stress, and reducing separation anxiety. In the case of livestock animals, there are the effects of improving meat quality and increasing milk production by reducing breeding stress even without the aid of drugs or the like. For leisure animals or sports animals, the excellent effects of imparting psychological stability to reduce the risk of accidents due to psychological stability, improving recovery from fatigue, and improving exercise ability can be brought about.

## Description

### Technical Field

The present disclosure relates to a device that stimulates the vagus nerve and, more particularly, to a vagus nerve stimulation device for animals that is developed to stimulate the vagus nerves of animals from the outside.

### Background Art

Currently, humans live together with many animals for various reasons. For example, there are companion animals that live with people, livestock animals from which meat or by-products are collected and used, leisure animals that are used in the leisure industry, and sports animals that are used for sports.

It is also necessary to ensure that these animals remain psychologically stable. Companion animals need psychological stability and stress relief, such as reducing aggression towards anything around the animals and relieving separation anxiety that occurs when the animals are separated for the work or travel of owners of the animals. In the case of livestock animals, effects such as improved meat quality and increased milk production can be achieved by reducing the stress of the bred animals. In the case of leisure and sports animals, due to psychological stability thereof, it is possible to obtain the effects of reducing accidents, improving exercise ability, and improving recovery from fatigue.

However, methods for stabilizing psychology of animals are limited and effectiveness thereof is uncertain. For example, administering drugs to animals may cause problems with the health of the animals, and in the case of livestock animals, leisure animals, and sports animals, the use of drugs is often restricted. Music is used in a harmless way, but effectiveness thereof is uncertain.

Accordingly, there is a growing demand for new methods that can induce certain psychological stability in various animals and do not cause health problems.

### Disclosure

### Technical Problem

the present disclosure has been made to solve the problems of the prior art described above, and is intended to propose a vagus nerve stimulation device for animals that is able to stabilize the psychology of an animal by stimulating the vagus nerve of the animal from the outside.

### Technical Solution

In order to accomplish the above objective, a vagus nerve stimulation device for animals the present disclosure includes an electrode configured to contact a skin of an animal to transmit electrical stimuli to the vagus nerve, and a power source for generating electrical stimuli transmitted to the electrode.

The device may further include a control part configured to control the electrical stimuli transmitted to the electrode.

The electrode may be formed on a collar worn around the neck of an animal, and an electrode part on which the electrode is formed may be configured to be attachable and detachable from the collar.

The electrode may be formed on a harness worn on a horse, and the electrode part on which the electrode is formed may be configured to be attachable and detachable from the harness.

The electrode may be formed on an ear tag attached to the ear of an animal, and the electrode part on which the electrode is formed may be configured to be attachable and detachable from the ear tag.

The electrode may be formed on an earmuff of a horse, and the electrode part on which the electrode is formed may be configured to be attachable and detachable from the earmuff.

The device may further include a controller having a control part that controls the electrical stimuli transmitted to the electrode, wherein the controller may be connected to the electrode by wire or wirelessly.

The device may further include a detection part configured to detect physical changes in response to electrical stimuli.

### Advantageous Effects

The stimulation device configured as described above has an excellent effect of stabilizing the psychological state of an animal by stimulating the vagus nerve of the animal from the outside of the animal.

For companion animals, the stimulation device has the effect of eliminating aggression, relieving stress, and reducing separation anxiety.

For livestock animals, the stimulation device has the effect of improving meat quality and increasing milk production by reducing breeding stress even without the aid of drugs, etc.

For leisure animals or sports animals, the stimulation device has the excellent effect of imparting psychological stability to reduce the risk of accidents and improve recovery from fatigue and exercise ability due to the psychological stability.

### Description of Drawings

FIG. 1 is a view illustrating a collar-type vagus nerve stimulation device for animals according to a first embodiment of the present disclosure.
FIG. 2 is a view illustrating a harness-type vagus nerve stimulation device for animals according to a second embodiment of the present disclosure.
FIG. 3 is a view illustrating an ear tag type vagus nerve stimulation device for animals according to a third embodiment of the present disclosure.
FIG. 4 is a view illustrating an earmuff-type vagus nerve stimulation device for animals according to a fourth embodiment of the present disclosure.
FIG. 5 is a view illustrating an earmuff-type vagus nerve stimulation device for animals according to a fifth embodiment of the present disclosure.

### <Description of the Reference Numerals in the Drawings>

| | | | |
|---|---|---|---|
| 10: | Collar | 20, 30: | Harness |
| 40: | Ear tag | 50: | Earmuff |
| 100: | Controller | 200: | Electrode part |

### Mode for Invention

Embodiments according to the present disclosure will be described in detail with reference to the attached drawings.

However, the embodiments of the present disclosure may be modified into several different forms, and the scope of the present disclosure is not limited to the embodiments described below. The shapes and sizes of elements in the drawings may be exaggerated for clearer explanation, and elements indicated by the same reference numerals in the drawings are the same elements.

In addition, throughout the specification, when a part is said to be "connected" to another part, this includes not only a case in which the parts are "directly connected" to each other but also a case in which the parts are "electrically connected" to each other with another element placed therebetween. Additionally, when a part is described to "include" or "have" a certain component, this means that the part does not exclude other components but may further include or have the other components, unless specifically stated to the contrary.

Additionally, terms such as "first" and "second", etc. are used to distinguish one component from another component, and the scope of the claims should not be limited by these terms. For example, a first component may be named a second component, and similarly, the second component may also be named the first component.

In animals, especially mammals, the vagus nerve, which is one of the cranial nerves, corresponds to the tenth cranial nerve. The vagus nerve originates from the brain and is distributed throughout the face, chest, and abdomen, and is a mixed nerve containing parasympathetic nerve fibers and is involved in the regulation of the parasympathetic nerves that act on the heart, lungs, and digestive tract. The vagus nerve has the longest and most complex structure among twelve pairs of cranial nerves, and includes both sensory nerve fibers and motor nerve fibers.

In the case of humans, vagal nerve stimulation devices for the treatment of epilepsy and depression are known to have been approved by the U.S. Food and Drug Administration (FDA), and target the cervical branches located in the neck for stimulation, and thus are inserted and installed inside the human body. Accordingly, a method of stimulating the brain by stimulating the vagus nerve is being studied in humans, but a technology to apply this method to animals has not been developed.

In the case of humans, the vagus nerve is connected to the brain through the ears and neck, and this is the same in animals. Accordingly, the inventors of the present disclosure developed a vagus nerve stimulation device for animals including an electrode that is installed outside a location at which the vagus nerve of an animal passes and applies electrical stimuli to the vagus nerve inside.

For example, when electrical stimuli are applied through an electrode installed to contact the skin of the neck or ear of an animal, the electrical stimuli are transmitted to the vagus nerve located inside the skin of the animal and stimulate the brain of the animal, thereby providing psychological stability to the animal.

The vagus nerve stimulation device for animals in the present disclosure may be configured in various ways depending on a position where the electrode is attached.

First, the vagus nerve stimulation device for animals may be configured such that the electrode is attached to the neck of an animal to apply electrical stimuli to the vagus nerve passing through the neck, and a collar-type vagus nerve stimulation device may be used as a structure for contacting the electrode to the neck.

Companion animals such as dogs and cats wear a collar worn around the neck thereof for various reasons, and thus an electrode may be formed on a collar for a general companion animal to be worn at all times, and a collar-type vagus nerve stimulation device may be worn on an animal only when necessary. When an electrode is formed on a collar that is worn all the time, it is preferable that a main body for applying electrical stimuli to the electrode and the collar are configured to be electrically connected to each other only when necessary.

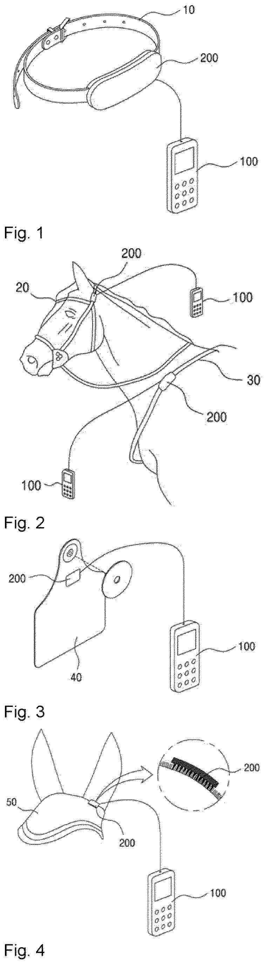

FIG. 1 is a view illustrating a collar-type vagus nerve stimulation device for animals according to a first embodiment of the present disclosure.

The collar-type vagus nerve stimulation device for animals according to this embodiment includes a controller 100 that controls electrical stimuli and an electrode part 200 having an electrode that contacts the skin of an animal to transmit electrical stimuli. The electrode part 200 is coupled to a collar 10 that is worn around the neck of an animal, and may be installed as a component separate from the collar as shown, or only the electrode is formed on the collar 10 and wire, etc. may be hidden therein. When the electrode part 200 is configured as a separate component from the collar, the electrode part 200 may be configured to be attachable and detachable from the collar 10, so that the electrode part 200 may be applied to a general collar. The electrode may be configured in a variety of ways to transmit electrical stimuli by contacting the skin of an animal, and may be configured by taking the fur of an animal into consideration. The controller 100 is connected to the electrode part 200 by wire or wirelessly and controls electrical stimuli applied from the electrode part 200. In the case of connection by wire, wire may be configured to be removed from the electrode part 200. Meanwhile, a power source is required to generate electrical stimuli, and the power source may be located in the electrode part 200 or the controller 100. When the power source is located in the controller 100, the controller 100 and the electrode part 200 are required to be connected to each other by wire, and when the power source is located in the electrode part 200, the controller 100 and the electrode part 200 are required to be connected wirelessly to transmit only a control signal. When the controller 100 is connected wirelessly, the controller 100 is not required to be a product specialized for the device of the present disclosure and may be an application of a smart device, etc.

A recreational animal such as a horse has a harness worn on the head and torso thereof, and since some components of the harness worn on the face to connect a rein thereto are located on the neck of a horse, an electrode may be formed on the harness to configure the vagus nerve stimulation device for animals, and since the breast band of the harness installed on the body of a horse for a saddle passes around the neck, the electrode may be formed on the breast band of the harness to configure the vagus nerve stimulation device for animals.

FIG. 2 is a view illustrating a harness-type vagus nerve stimulation device for animals according to a second embodiment of the present disclosure.

The harness-type vagus nerve stimulation device for animals according to this embodiment includes a controller 100 that controls electrical stimuli and an electrode part 200 having an electrode that contacts the skin of an animal to transmit electrical stimuli. The electrode part 200 is coupled to a harness 20 worn on the head of a horse or a harness 30 worn on the body thereof, and may be installed as a separate component from the harness as shown, or only the electrodes are formed on the harnesses 20 and 30, and wire, etc. may be hidden therein. When the electrode part 200 is configured as a separate component from the harness, the electrode part 200 may be configured to be attachable and detachable from the harnesses 20 and 30, so that the electrode part 200 may be applied to a general harness. The electrode may be configured in a variety of ways to transmit electrical stimuli by contacting the skin of an animal, and may be configured by taking the fur of an animal into consideration. The controller 100 is connected to the electrode part 200 by wire or wirelessly and controls electrical stimuli applied from the electrode part 200. In the case of connection by wire, wire may be configured to be removed from the electrode part 200. Meanwhile, a power source is required to generate electrical stimuli, and the power source may be located in the electrode part 200 or the controller 100. When the power source is located in the controller 100, the controller 100 and the electrode part 200 are required to be connected to each other by wire, and when the power source is located in the electrode part 200, the controller 100 and the electrode part 200 are required to be connected wirelessly to transmit only a control signal. When the controller 100 is connected wirelessly, the controller 100 is not required to be a product specialized for the device of the present disclosure and may be an application of a smart device, etc.

In the case of a livestock animal such as a cow, a general collar may be worn thereon, but a fixing collar is often used to secure the animal during work. An electrode may be formed on the general collar, or on the fixing collar. When forming the electrode on the fixing collar, electrical stimuli are applied to the vagus nerve while an animal is fixed for work, thereby reducing stress the animal receives during the work.

Next, the vagus nerve stimulation device for animals may be configured such that an electrode is attached to the ear to apply electrical stimuli to the vagus nerve passing through the ear of an animal.

There are two ways to attach the electrode to the ear: a method of fixing the electrode in the form of a clamp without piercing the ear, and a method of fixing the electrode in the form of an ear tag that pierces the ear.

The clamp-type vagus nerve stimulation device for animals has the advantage of being attachable and detachable from the ear of an animal, but there is a risk of detachment due to the movement of the animal, and an ear tag type vagus nerve stimulation device for animals has the disadvantage of being fixed to the ear of an animal.

Since livestock such as cows often have ear tags attached thereto, an ear tag type vagus nerve stimulation device for animals is suitable.

FIG. 3 is a view illustrating an ear tag type vagus nerve stimulation device for animals according to a third embodiment of the present disclosure.

The ear tag type vagus nerve stimulation device for animals according to this embodiment includes the controller 100 that controls electrical stimuli and the electrode part 200 including an electrode that contacts the skin of an animal to transmit electrical stimuli. The electrode part 200 may be installed on a part of the ear tag 40 attached to the ear of an animal that contacts the skin of the animal and may be installed as a separate component from the ear tag as shown, or only the electrode may be formed on the ear tag 40 and wire, etc. may be hidden. When the electrode part 200 is configured as a separate component from the ear tag, the electrode part 200 may be configured to be attachable and detachable from the ear tag 40, so that the electrode part 200 may be applied to a general ear tag. The electrode may be configured in a variety of ways to transmit electrical stimuli by contacting the skin of an animal, and may be configured by taking the fur of an animal into consideration. The controller 100 is connected to the electrode part 200 by wire or wirelessly and controls electrical stimuli applied from the electrode part 200. In the case of connection by wire, wire may be configured to be removed from the electrode part 200. Meanwhile, a power source is required to generate electrical stimuli, and the power source may be located in the electrode part 200 or the controller 100. When the power source is located in the controller 100, the controller 100 and the electrode part 200 are required to be connected to each other by wire, and when the power source is located in the electrode part 200, the controller 100 and the electrode part 200 are required to be connected wirelessly to transmit only a control signal. When the controller 100 is connected wirelessly, the controller 100 is not required to be a product specialized for the device of the present disclosure and may be an application of a smart device, etc.

In the case of a companion animal, the ear tag is not attached, and in the case of a clamp-type vagus nerve stimulation device, there is a high possibility of separation, so the vagus nerve stimulation device may be selectively worn only when necessary to stimulate the vagus nerve.

Meanwhile, a riding horse uses a fly mask or fly veil in the form of an earmuff that covers the ears to protect against flying insects, and since an earmuff can contact the ears, a structure in which an electrode is formed on the earmuff is possible.

FIG. 4 is a view illustrating an earmuff-type vagus nerve stimulation device for animals according to a fourth embodiment of the present disclosure.

The earmuff-type vagus nerve stimulation device for animals according to this embodiment includes a controller 100 that controls electrical stimuli and an electrode part 200 having an electrode that contacts the skin of an animal to transmit electrical stimuli. The electrode part 200 is installed on a part of the earmuff 50 connected to the scalp and ear pinna of a horse, which are parts that contact the skin of the animal, and may be installed as a separate component from the earmuff as shown, or only the electrode may be formed on the earmuff 50 and wire, etc. may be hidden. When the electrode part 200 is configured as a separate component from the earmuff, the electrode part 200 may be configured to be attachable and detachable from the earmuff 50, so that the electrode part 200 may be applied even to a general earmuff. The electrode may be configured in a variety of ways to transmit electrical stimuli by contacting the skin of an animal, and may be configured by taking the fur of an animal into consideration. For example, as shown in the enlarged cross-sectional view of FIG. 4, multiple electrodes in the form of protruding protrusions are formed in the electrode part 200, and by configuring the electrodes in the form of protrusions, the electrodes may contact the skin of a horse even in hairy areas. The controller 100 is connected to the electrode part 200 by wire or wirelessly, and controls electrical stimuli applied from the electrode part 200. In the case of connection by wire, wire may be configured to be removed from the electrode part 200. Meanwhile, a power source is required to generate electrical stimuli, and the power source may be located in the electrode part 200 or the controller 100. When the power source is located in the controller 100, the controller 100 and the electrode part 200 are required to be connected to each other by wire, and when the power source is located in the electrode part 200, the controller 100 and the electrode part 200 are required to be connected wirelessly to transmit only a control signal. When the controller 100 is connected wirelessly, the controller 100 is not required to be a product specialized for the device of the present disclosure and may be an application of a smart device, etc.

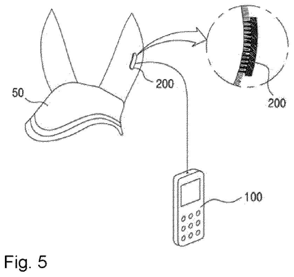

FIG. 5 is a view illustrating an earmuff-type vagus nerve stimulation device for animals according to a fifth embodiment of the present disclosure.

The earmuff-type vagus nerve stimulation device for animals of this embodiment includes a controller 100 that controls electrical stimuli and an electrode part 200 having an electrode that contacts the skin of an animal to transmit electrical stimuli. The electrode part 200 may be installed on a part of the earmuff 50 that contacts the skin of an animal and may include a plurality of electrodes, wherein the electrode part 200 is installed to be in contact with the auricle of a horse. Accordingly, other than difference in the installation location of the electrode part 200, the earmuff-type vagus nerve stimulation device for animals according to the fifth embodiment is the same as the earmuff-type vagus nerve stimulation device for animals according to the fourth embodiment described above, and thus detailed description thereof is omitted.

Meanwhile, the vagus nerve stimulation device for animals of the present disclosure may further include a sound generation part.

When acoustic or sound wave stimuli, together with electrical stimuli, are applied to the vagus nerve, it is possible to obtain the effect of improving a vagus nerve stimulation effect or activating the brain. Accordingly, the vagus nerve stimulation device for animals may further include the sound generation part in addition to the electrodes.

In general, animals have more developed hearing than humans, and thus acoustic stimuli may be applied thereto by adding the sound generation part to the vagus nerve stimulation device that is worn on the ear, and furthermore, even by adding the sound generation part to the vagus nerve stimulation device worn around the neck, acoustic stimuli, along with electrical stimuli, may be applied to the vagus nerve.

Furthermore, the vagus nerve stimulation device for animals of the present disclosure may further include a detection part that detects changes in the body of an animal.

Unlike humans who can communicate, animals have difficulty communicating directly, so it is difficult to receive feedback on the intensities of electrical stimuli from the animals. However, the detection part that detects the response of the body to excessive electrical stimuli may be added to control the electrical stimuli.

The detection part may select a response to an excessive electrical stimulus among various body changes through which feedback can be received. For example, the detection part that detects changes in brain waves, a pulse, a body temperature, and electromyogram, etc. may be applied.

In particular, it is possible to control an electrical stimulus by measuring the electromyogram of a predetermined muscle and feeding back a response to the intensity of the electrical stimulus. The detection part that measures electromyogram may be configured in various forms depending on a muscle to be measured, and the muscle to be measured may be selected depending on the type of an animal.

The present disclosure has been described above through exemplary embodiments, but the above-described embodiments are merely illustrative descriptions of the technical idea of the present disclosure, and those skilled in the art will understand that the embodiments may be variously changed without departing from the technical idea of the present disclosure. Accordingly, the scope of protection of the present disclosure should be interpreted on the basis of the matters stated in the claims, not the specific embodiments, and all technical ideas within the equivalent scope should be interpreted as being included in the scope of the claims of the present disclosure.

## Claims

1. A vagus nerve stimulation device for animals, the device comprising:
an electrode configured to contact a skin of an animal to transmit electrical stimuli to the vagus nerve; and
a power source for generating electrical stimuli transmitted to the electrode.

2. The device of claim 1, wherein the electrode is formed on one of a collar worn around a neck of an animal, a harness worn on a horse, an ear tag attached to an ear of an animal, and an earmuff of a horse.

3. The device of claim 2, wherein an electrode part on which the electrode is formed is attachable and detachable to one of the collar, the harness, the ear tag, and the earmuff.

4. The device of claim 1, further comprising:
a control part configured to control the electrical stimuli transmitted to the electrode.

5. The device of claim 2, further comprising:
a controller having a control part that controls the electrical stimuli transmitted to the electrode,
wherein the controller is connected to the electrode by wire or wirelessly.

6. The device of claim 1, further comprising:
a detection part configured to detect physical changes in response to electrical stimuli.
